# EUROPEAN PATENT SPECIFICATION

(11) **EP 4 027 974 B1**
(45) Date of publication and mention of the grant of the patent: **26.02.2025**
(21) Application number: 20781115.9
(22) Date of filing: 11.09.2020
(51) Int. Cl.: A61K 9/00, A61K 9/08, A61J 1/00, A61K 31/00, A61K 47/18, A61J 1/10, A61K 31/69

(54) **PHARMACEUTICAL COMPOSITION CONTAINING P-BORONOPHENYLALANINE**
PHARMAZEUTISCHE ZUSAMMENSETZUNG, DIE P-BORONOPHENYLALANIN ENTHÄLT
COMPOSITION PHARMACEUTIQUE CONTENANT DE LA P-BORONOPHENYLALANINE

(30) Priority: 12.09.2019 JP 2019165835
(43) Date of publication of application: 20.07.2022
(60) Divisional application: 25152531.7
(73) Proprietor: Stella Pharma Corporation, Osaka-shi, Osaka 541-0043 (JP)
(72) Inventor: IGUCHI, Yoshiya, Osaka-shi, Osaka 541-0043 (JP); KATAKUSE, Yoshimitsu, Osaka-shi, Osaka 541-0043 (JP); NAKASHIMA, Hideki, Osaka-shi, Osaka 541-0043 (JP)
(74) Representative: Hoffmann Eitle
(86) International application number: PCT/JP2020/034391
(87) International publication number: WO 2021/049599

(56) References cited:
- JP-A- 2008 100 925
- JP-A- 2013 173 804

## Description

### [Technical Field]

The present invention relates to a pharmaceutical composition.

### [Background Art]

Boron neutron capture therapy (BNCT) is known as a cancer treatment method in which radioactive isotopes are used. Boron neutron capture therapy is a treatment method including incorporating a boron compound containing a boron-10 isotope (¹⁰B; hereinafter, also referred to as 10B) into cancer cells and applying a low energy neutron beam (for example, thermal neutrons) thereto to locally destroy the cancer cells through a nuclear reaction that occurs inside the cancer cells. In this treatment method, boron compounds, for example, p-boronophenylalanine (hereinafter, also referred to as BPA) that are boron compounds containing 10B which can be selectively accumulated in the cells of cancer tissue are used.

A method for injecting a liquid composition containing BPA is used as a method for administering BPA to a living body. Since BPA has low solubility, a formulation method for homogenizing a liquid composition containing BPA has been studied. For example, Patent Document 1 discloses a liquid composition which contains BPA and sorbitol and in which a ratio of the content of sorbitol to the content of BPA is within a range of 0.9 or more and 2 or less as a molar ratio as a liquid composition containing BPA having excellent solubility and stability.

### [Citation List]

### [Patent Literature]

[PTL 1]
   Patent Publication JP-A-2009-51766
[PTL2]
   JP2013173804A relates to a luiquid pharmaceutical composition containing p-borophenylalanine, sorbitol, and a sulfite, wherein the content of sorbitol is in a molar ratio of 0.9 to 2 with respect to the content of p-borophenylalanine.
[PTL3]
   JP2008100925A relates to a pharmaceutical composition comprising p-borophenylalanine and meglumine.

### [Summary of Invention]

### [Technical Problem]

A liquid composition containing BPA as an injection or the like may be prepared immediately before administration. However, for efficient administration to a patient, it is conceivable that a BPA-containing liquid composition is distributed and stored in a form in which the BPA-containing liquid composition prepared in advance is accommodated in a packaging material, that is, an infusion bag or the like. At this time, it is required for the BPA-containing liquid composition prepared in advance to keep components such as BPA in the liquid composition stable until the liquid composition is administered to a patient.

In addition, a heat treatment is performed for sterilization when producing an infusion bag. At this time, BPA in a liquid composition is easily changed by heat, which is problematic.

With the foregoing in view, an object of the present invention is to provide a pharmaceutical composition in which components such as BPA are kept stable even during heating due to sterilization or the like or long-term storage.

### [Solution to Problem]

The present inventors have conducted extensive studies, and as a result, they have found that a pharmaceutical composition of BPA in which deterioration of components in a liquid composition containing BPA against heating during sterilization is inhibited and which has excellent long-term storage stability can be obtained through a predetermined production method, thus leading to realization of the present invention. In addition, the present inventors have found that it is possible to obtain a formulation having excellent long-term storage stability by controlling the amount of dissolved oxygen in the liquid composition containing BPA, thus leading to completion of the present invention.

That is, the present invention is as defined in the claims. The following embodiments may be used to further understand the present invention.
(1) A pharmaceutical composition including a liquid composition containing p-boronophenylalanine, the liquid composition being accommodated in a packaging material and satisfying the following Condition I or Condition II:
   Condition I: the liquid composition comprises substantially no antioxidant, i.e. it comprises components of antioxidant less than 0.001 w/v%, and has a concentration of dissolved oxygen in the liquid composition of 3.5 ppm or less.
   Condition II: the liquid composition comprises an antioxidant, and has a concentration of dissolved oxygen in the liquid composition of 3.0 ppm or less.
(2) The pharmaceutical composition according to (1), in which the liquid composition further comprises 0.75 mass% or less of tyrosine.
(3) The pharmaceutical composition according to (1) or (2), in which a tyrosine content in the liquid composition after the pharmaceutical composition is stored at 40°C/75%RH for 2 weeks is 0.40 mass% or less under Condition I or 1.20 mass% or less under Condition II.
(4) The pharmaceutical composition according to any one of (1) to (3), in which the antioxidant comprises at least one selected from the group consisting of sodium pyrosulfite, sodium hydrogen sulfite, α-tocopherol, ascorbic acid, ascorbyl palmitate, butylhydroxyanisole, butylhydroxytoluene, monothioglycerol, potassium metabisulfite, propionic acid, propyl gallate, sodium ascorbate, sodium iodide, sodium nitrite, sodium sulfite, and sodium thiosulfate.
(5) The pharmaceutical composition according to any one of (1) to (4), in which the packaging material is a bag-shaped packaging material.
(6) The pharmaceutical composition according to any one of (1) to (5), in which a material of the packaging material comprises a thermoplastic resin.
(7) The pharmaceutical composition according to (6), in which the thermoplastic resin comprises at least one selected from the group consisting of polyethylene resins, polypropylene resins, polyvinyl chloride resins, polyvinylidene chloride resins, polystyrene resins, polyvinyl acetate resins, polyurethane resins, polytetrafluoroethylene (PTFE) resins, acrylonitrile butadiene styrene resins, and acrylic resins.
(8) The pharmaceutical composition according to any one of (1) to (7), in which the packaging material is an infusion bag.
(9) In a reference embodiment, not being part of the invention, a method for producing a pharmaceutical composition which comprises a liquid composition containing p-boronophenylalanine and is accommodated in a packaging material, the method including:
   a step I of accommodating the liquid composition containing p-boronophenylalanine in the packaging material; and
   a step II of heat-sterilizing the liquid composition accommodated in the packaging material under an inert gas atmosphere.
(10) The production method according to (9), in which the liquid composition comprises an antioxidant.
(11) The production method according to (10), in which the antioxidant comprises at least one selected from the group consisting of sodium pyrosulfite, sodium hydrogen sulfite, α-tocopherol, ascorbic acid, ascorbic acid palmitate, butylhydroxyanisole, butylhydroxytoluene, monothioglycerol, potassium metabisulfite, propionic acid, propyl gallate, sodium ascorbate, sodium iodide, sodium nitrite, sodium sulfite, and sodium thiosulfate.
(12) The production method according to any one of (9) to (11), in which the packaging material is a bag-shaped packaging material.
(13) The production method according to any one of (9) to (12), in which a material of the packaging material comprises a thermoplastic resin.
(14) The production method according to (13), in which the thermoplastic resin comprises at least one selected from the group consisting of polyethylene resins, polypropylene resins, polyvinyl chloride resins, polyvinylidene chloride resins, polystyrene resins, polyvinyl acetate resins, polyurethane resins, polytetrafluoroethylene resins, acrylonitrile butadiene styrene resins, and acrylic resins.
(15) The production method according to any one of (9) to (14), in which the packaging material is an infusion bag.
(16) The production method according to any one of (9) to (15), in which the step I is performed under an inert gas atmosphere.

### [Advantageous Effects of Invention]

According to the present invention, it is possible to stably store a pharmaceutical composition, in which a liquid composition containing BPA is accommodated in a packaging material, for a long period of time. In addition, according to the present invention, it is possible to provide a pharmaceutical composition while suppressing the denaturation of the liquid composition containing BPA and can be stably stored for a long period of time.

### [Brief Description of Drawings]

[Fig. 1]

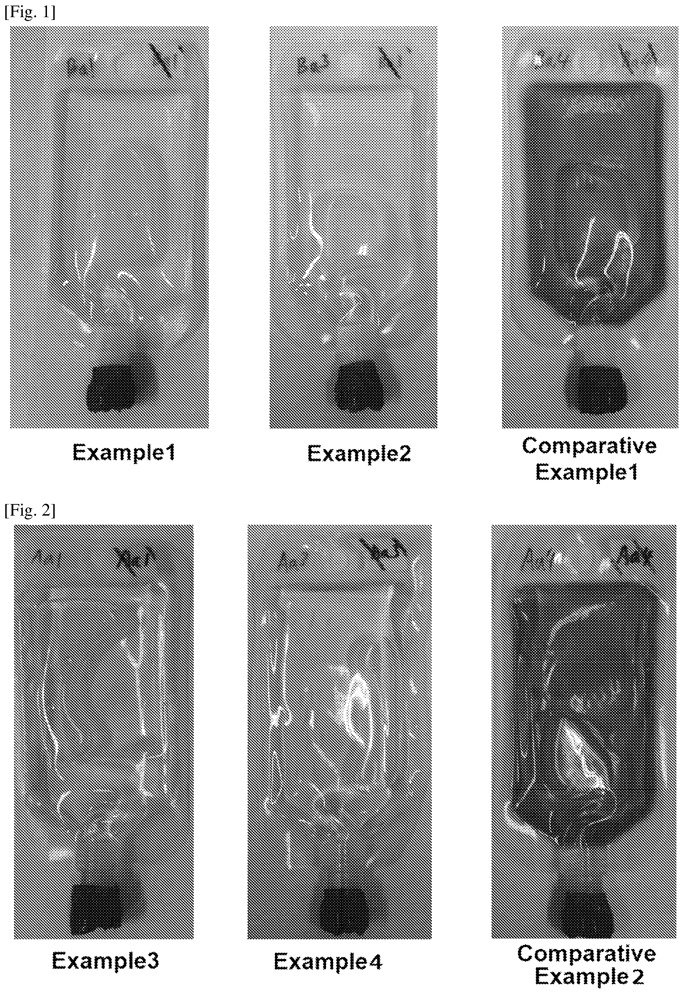

   FIG. 1 shows photographs of pharmaceutical compositions of Examples 1 and 2 and Comparative Example 1.
[Fig. 2]
   FIG. 2 shows photographs of pharmaceutical compositions of Examples 3 and 4 and Comparative Example 2.
[Fig. 3]

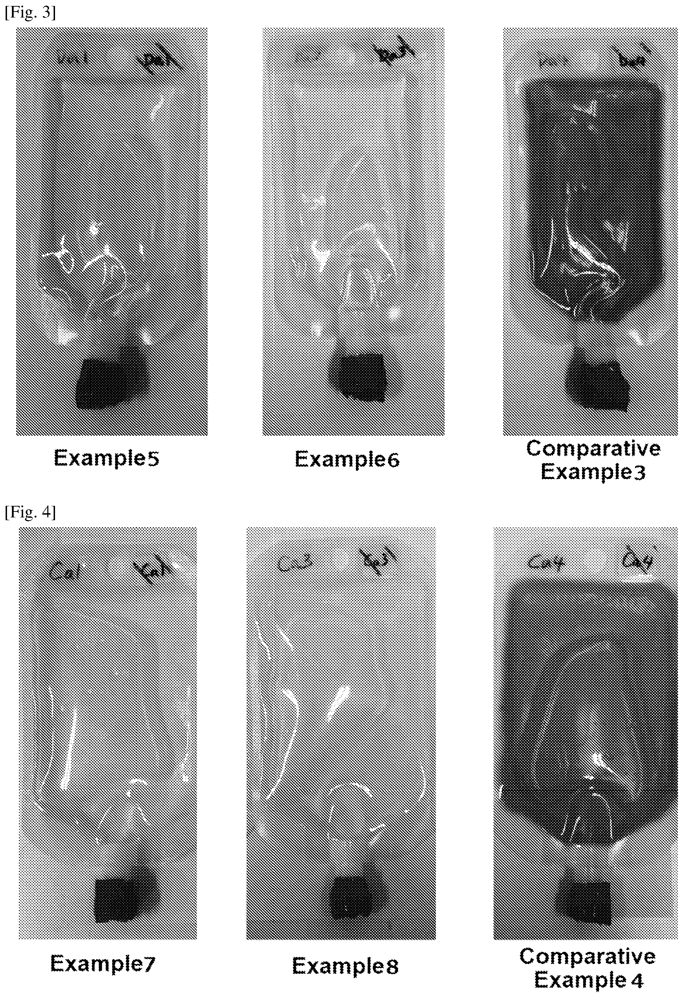

   FIG. 3 shows photographs of pharmaceutical compositions of Examples 5 and 6 and Comparative Example 3.
[Fig. 4]
   FIG. 4 shows photographs of pharmaceutical compositions of Examples 7 and 8 and Comparative Example 4.

### [Description of Embodiments]

Hereinafter, an embodiment of the present invention will be described in detail.

A pharmaceutical composition of the present invention is a pharmaceutical composition which comprises a liquid composition containing p-boronophenylalanine and in which the liquid composition is accommodated in a packaging material according to claim 1. Here, the pharmaceutical composition of the present invention can also be said to be, in other words, a kit or a pharmaceutical formulation which comprises a liquid composition containing p-boronophenylalanine and a packaging material accommodating the liquid composition. The kit or the pharmaceutical formulation in the present specification refers to a set comprising at least a liquid composition to be administered to a subject and a packaging material accommodating the liquid composition. In addition, the pharmaceutical composition of the present invention satisfies the following Condition I or Condition II:
Condition I: the liquid composition comprises substantially no antioxidant, and a concentration of dissolved oxygen in the liquid composition is 3.5 ppm or less;
Condition II: the liquid composition comprises an antioxidant, and a concentration of dissolved oxygen in the liquid composition is 3.0 ppm or less.

In addition, the present disclosure relates to a method for producing a pharmaceutical composition which comprises a liquid composition containing p-boronophenylalanine and accommodated in a packaging material.

The production method of the present disclosure includes: a step I of accommodating the liquid composition containing p-boronophenylalanine in a packaging material; and a step II of heat-sterilizing the liquid composition accommodated in the packaging material under an inert gas atmosphere.

The present inventors have found that storage of a liquid composition containing BPA for a long period of time causes coloration of the liquid and a change in the liquid composition. In addition, the present inventors have found that, in a case where dissolved oxygen is present in a liquid composition containing BPA, a change in the liquid composition is caused during storage of the liquid composition, and stability of BPA is not maintained. Specifically, it has become clear that BPA in a liquid composition turns into tyrosine or the like as the amount of dissolved oxygen increases.

The present inventors have conducted extensive studies on such findings, and as a result, they have found that controlling the concentration of dissolved oxygen in a liquid composition containing BPA such that it is a predetermined level can inhibit changes in the liquid composition, prevent the liquid from becoming colored when the liquid is stored for a long period of time, and can inhibit changes in BPA.

In addition, they have found that the suppression of the concentration of dissolved oxygen such that it is a low level is achieved through heat-sterilizing under an inert gas atmosphere a kit in which a liquid composition containing BPA is accommodated.

BPA is an analog of amino acids such as phenylalanine or tyrosine, and is incorporated into cancer cells through an amino acid transporter such as LAT1. An amino acid transporter has an inherent function of incorporating natural-type amino acids into cancer cells. Therefore, in competition between BPA which is a non-natural-type amino acid and natural-type amino acids for incorporation into cancer cells, a natural-type amino acid is more easily incorporated into cancer cells. That is, it is possible to efficiently incorporate BPA into cancer cells by reducing the content of tyrosine in a pharmaceutical composition.

In addition, coloration of the pharmaceutical composition of the present invention is caused by a change in components in the composition, and it is desirable to suppress a change in the components in the pharmaceutical composition to maintain the potency.

In the pharmaceutical composition of the present invention, it is possible to maintain the potency by suppressing generation of tyrosine from BPA and coloration even if the pharmaceutical composition is stored for a long period of time. Similarly, in the production method of the present invention, decomposition of BPA due to heating in a heat sterilization step can be suppressed and the concentration of dissolved oxygen can be reduced, and therefore, the potency of an obtained pharmaceutical composition can be maintained.

### (Pharmaceutical Composition)

The pharmaceutical composition of the present invention can be used for boron neutron capture therapy (BNCT). The pharmaceutical composition of the present invention can be in dosage forms such as nasal drops, oral cavity preparations, vaginal preparations, suppositories, and injections. That is, the pharmaceutical composition of the present invention may be in any form of oral administration of a liquid agent or the like or parenteral administration of injections for arterial injection, intravenous injection, intramuscular injection, and the like, suppositories, or percutaneous formulations. A form of administration to a central vein using a catheter or the like is also included in intravenous injection. In addition, direct administration to tissue, for example, administration to cancer tissue is also included in parenteral administration.

Examples of parenteral administration include intravenous administration, intraarterial administration, intramuscular administration, subcutaneous administration, intratissue administration, intranasal administration, intradermal administration, intracerebral administration, intrarectal administration, intravaginal administration, and intraperitoneal administration. Among these, injections are preferably used, and instillation is more preferably performed.

Target diseases for BNCT are not particularly limited, but examples thereof include solid cancer and cancer (epithelial tumor) arising from epithelial cells. Specific examples thereof include skin cancer including melanoma or the like, lung cancer, breast cancer, stomach cancer, colon cancer, uterine cancer, ovarian cancer, head and neck cancers (such as laryngeal cancer, pharyngeal cancer, and tongue cancer). In addition, the target diseases may be sarcomas originating from non-epithelial cells, and examples of the sarcomas include osteosarcomas, refractory osteosarcomas, rhabdomyosarcomas, leiomyosarcomas, fibrosarcomas, liposarcomas, and haemosarcomas. Furthermore, other examples of the target diseases include brain tumors such as gliomas, primary malignant lymphomas of the central nervous system, meningiomas, pituitary adenoma, schwannoma, and craniopharyngiomas. Initial cancers, single cancers, cancers that have spread to individual organs, metastatic cancers, and refractory cancers can be targets for BNCT.

### (Liquid Composition Containing BPA)

The liquid composition containing BPA of the present invention is not particularly limited as long as it is a liquid composition containing at least BPA. The liquid composition of the present invention contains BPA and a solvent. The liquid composition may be a suspension of BPA, a solution of BPA, or a colloidal solution of BPA, but is preferably a solution of BPA. It is preferable that the liquid composition be homogeneously dissolved in the composition.

The BPA of the present invention is p-boronophenylalanine, and may be an L-form, a D-form, or a racemate containing both an L-form and a D-form. BPA preferably contains the L-form. The BPA of the present invention preferably contains the boron-10 isotope (¹⁰B). Specifically, the BPA of the present invention preferably contains a compound in which boron in a boronic acid moiety of BPA is ¹⁰B. Here, the proportion of a compound in which boron in a boronic acid moiety of BPA is ¹⁰B in BPA used in the present invention based on the total amount of BPA generally may be 50 mass% or more, preferably 75 mass% or more, more preferably 80 mass% or more, still more preferably 85 mass% or more, still more preferably 90 mass% or more, still more preferably 95 mass% or more, and particularly preferably 99 mass% or more. The upper limit of the above-described proportion is generally 100 mass%. Commercially available products may be used for the BPA and the BPA may be synthesized through well-known methods (for example, H. R. Synder, A. J. Reedy, W. M. J. Lennarz, J. Am. Chem. Soc., 1958, 80, 835: C. Malan, C. Morin, SYNLETT, 1996, 167: U.S. Patent No. 5157149, Patent Publication JP-A-2000-212185, and Japanese Patent No. 2979139).

Examples of the solvent contained in the liquid composition of the present invention include water and alcohols. These may be used alone or in combination of two or more thereof. Among these, water is preferable. Aqueous solvents such as injection water, physiological saline, and a Ringer's solution may be used as water used as the solvent. In addition, vegetable oils such as olive oil, sesame oil, cottonseed oil, and corn oil, and oily solvents such as propylene glycol may be included as the solvent.

The content of BPA in the liquid composition of the present invention may be appropriately determined depending on the types of components to be formulated, but is generally 2.0 W/V% or more and 8.0 W/V% or less. In the case where the content of BPA is 2.0 W/V% or more, the liquid composition tends to be able to be administered to a patient without burden during a treatment. In the case where the content of BPA is 8.0 W/V% or less, the liquid composition tends to be able to be kept stable in a solution state and the osmotic pressure ratio tends to be able to be controlled such that it is within a range suitable as an injection.

The liquid composition of the present invention preferably contains sorbitol from the viewpoints of homogeneously dissolving BPA in the liquid composition and thereby enhancing the stability of the liquid composition. Sorbitol serves as a solubilizing agent for BPA. D-sorbitol which is currently approved for use in pharmaceutical products and of which safety has been confirmed is preferably used as the sorbitol, but the present invention is not limited thereto. In addition to the D-form, the L-form or a mixture of the L-form and the D-form can be used for the sorbitol.

In addition to sorbitol, fructose (in addition to the D-form, the L-form or a mixture of the L-form and the D-form can also be used), xylitol (in addition to the D-form, the L-form or a mixture of the L-form and the D-form can also be used), polyethylene glycol, propylene glycol, mannitol (in addition to the D-form, the L-form or a mixture of the L-form and the D-form can also be used), benzyl benzoate, ethanol, trisaminomethane, cholesterol, triethanolamine, sodium carbonate, or sodium citrate can also be used as the solubilizing agent, for example.

The content of solubilizing agent used in the liquid composition of the present invention may be appropriately adjusted depending on the amount of other additives formulated, but is, in terms of molar ratio based on the amount of BPA, preferably within a range of 0.9 or more and 2 or less and more preferably within a range of 1.1 or more and 1.5 or less. By setting the content of solubilizing agent to be 0.9 or more in terms of molar ratio based on the amount of BPA, the tolerance range of pH with respect to solubility tends to be able to be widened and precipitation of BPA in the body tends to be able to be suppressed. By setting the content of solubilizing agent to be 2 or less in terms of molar ratio based on the amount of BPA, the osmotic pressure ratio tends to be reduced and the solubilizing agent tends to work sufficiently.

The pH of the liquid composition of the present invention is preferably an almost neutral pH from the viewpoint of safety during administration to a living body. Specifically, the pH is preferably within a range of 6.5 or more and 8.0 or less and more preferably within a range of 7.4 or more and 7.8 or less. The pH can be controlled with a buffer agent, a pH adjuster (such as hydrochloric acid or sodium hydrogen carbonate), and the like suitably used in the formulation field.

The osmotic pressure ratio of the liquid composition of the present invention is not particularly limited, but is, in comparison with physiological saline, preferably within a range of 1 or more and 2 or less and more preferably within a range of 1.1 or more and 1.5 or less. By setting the osmotic pressure ratio to be within a range of 1 or more and 2 or less, reduction in pain or shortening of administration time tends to be able to be achieved when the pharmaceutical composition is administered to a living body as an injection.

Various metal ions which may be appropriately contained in a living body may be contained in the liquid composition of the present invention in order to achieve stability in vivo and in vitro. Suitable examples of metal ions include sodium ions. The concentration of metal ions is not particularly limited, but is generally within a range of 130 mEq/L or more and 160 mEq/L or less. In the case where the concentration of metal ions is within a range of 130 mEq/L or more and 160 mEq/L or less, the concentration of metal ions tends to be close to a range of a Na ion concentration in a body fluid and the electrolyte balance between an intracellular fluid and an extracellular fluid tends to be able to be maintained.

The pharmaceutical composition of the present invention satisfies Condition I or Condition II as described above.

### (Condition I)

Condition I is a condition that the liquid composition comprises substantially no antioxidant, and a concentration of dissolved oxygen in the liquid composition is 3.5 ppm or less.

Here, the expression that "the liquid composition comprises substantially no antioxidant" means a state where no antioxidant has been added to the liquid composition but a trace amount of an antioxidant may be contained in the liquid composition. The concentration of components of the antioxidant in the liquid composition is less than 0.001 W/V% and preferably 0 W/V%.

The concentration of dissolved oxygen under Condition I is 3.5 ppm or less. By setting the concentration of dissolved oxygen under Condition I to be 3.5 ppm or less, coloration can be suppressed during long-term storage of the pharmaceutical composition of the present invention and generation of tyrosine due to BPA decomposing can be suppressed. The concentration of dissolved oxygen under Condition I is preferably 3.0 ppm or less and more preferably 2.8 ppm or less.

The lower limit of the concentration of dissolved oxygen under Condition I is ideally 0 ppm, but may be greater than 0 ppm, may be 1.0 ppm or more, and may be 1.5 ppm or more since the coloration and the generation of tyrosine can be sufficiently suppressed even at such concentrations.

### (Condition II)

Condition II is a condition that the liquid composition comprises an antioxidant, and a concentration of dissolved oxygen in the liquid composition is 3.0 ppm or less. Here, the concentration of components of the antioxidant in the liquid composition is preferably 0.001 W/V% or more and 0.600 W/V% or less and more preferably 0.005 W/V% or more and 0.100 W/V% or less of the liquid composition.

The concentration of dissolved oxygen under Condition II is 3.0 ppm or less. By setting the concentration of dissolved oxygen under Condition II to be 3.0 ppm or less, coloration can be suppressed during long-term storage of the pharmaceutical composition of the present invention and generation of tyrosine due to BPA decomposing can be suppressed. The concentration of dissolved oxygen under Condition II is preferably 2.8 ppm or less and more preferably 2.5 ppm or less.

The lower limit of the concentration of dissolved oxygen under Condition II is ideally 0 ppm, but may be greater than 0 ppm, may be 0.5 ppm or more, and may be 1.0 ppm or more since the coloration and the generation of tyrosine can be sufficiently suppressed even at such concentrations.

The concentration of dissolved oxygen in the pharmaceutical composition of the present invention can be controlled such that it is within the above-described predetermined values through, for example, a method in which a step of accommodating a liquid composition in which a packaging material is filled with the liquid composition and is sealed is performed under an inert gas atmosphere during production of the pharmaceutical composition, specifically a method in which a step of heat-sterilizing the kit obtained through the accommodation step is performed under an inert gas atmosphere. Specifically, the concentration of dissolved oxygen can be controlled through the following method for producing a pharmaceutical composition.

The concentration of dissolved oxygen in the pharmaceutical composition of the present invention can be measured through a method described in the examples.

As described above, tyrosine is sometimes generated due to BPA decomposing at the time when the pharmaceutical composition of the present invention is heat-sterilized, and therefore, there is a concern that drug efficacy may deteriorate. The liquid composition of the pharmaceutical composition of the present invention under both Condition I and Condition II may contain 0.75 mass% or less of tyrosine, 0.60 mass% or less of tyrosine, or 0.50 mass% or less of tyrosine.

In addition, the content of tyrosine in the liquid composition of the pharmaceutical composition of the present invention is preferably 0.25 mass% or less in the liquid composition under Condition I. The content of tyrosine in the liquid composition of the pharmaceutical composition of the present invention is preferably 0.56 mass% or less in the liquid composition under Condition II. By setting the content of tyrosine under both Condition I and Condition II to be within the above-described ranges, coloration tends to be suppressed during long-term storage of the pharmaceutical composition of the present invention and generation of tyrosine due to BPA decomposing tends to be able to be suppressed.

The content of tyrosine in the liquid composition after the pharmaceutical composition of the present invention is stored for two weeks at 40°C/75%RH which corresponds to long-term storage conditions is preferably 0.40 mass% or less under Condition I or preferably 1.20 mass% or less under Condition II. In addition, the content of tyrosine in the liquid composition after the pharmaceutical composition in which the above-described liquid composition further contains 0.75 mass% or less of tyrosine, 0.60 mass% or less of tyrosine, or 0.50 mass% or less of tyrosine is stored for two weeks at 40°C/75%RH is preferably within the above-described ranges.

The content of tyrosine in the pharmaceutical composition after the pharmaceutical composition is stored for two weeks at 40°C/75%RH can be regarded as one of indicators of long-term stability, and stable storage of the pharmaceutical composition tends to be exhibited by the content thereof being within the above-described ranges.

The content of tyrosine in the liquid composition of the pharmaceutical composition of the present invention before the pharmaceutical composition is stored at 40°C/75%RH (at a point in time of starting the storage) is preferably 0.25 mass% or less with respect to the liquid composition under Condition I, and the content thereof after the pharmaceutical composition is stored for 2 weeks at 40°C/75%RH is preferably 0.40 mass% or less with respect to the liquid composition under Condition I.

The content of tyrosine in the liquid composition of the pharmaceutical composition of the present invention before the pharmaceutical composition is stored at 40°C/75%RH (at a point in time of starting the storage) is preferably 0.56 mass% or less with respect to the liquid composition under Condition II, and the content thereof after the pharmaceutical composition is stored for two weeks at 40°C/75%RH is preferably 1.20 mass% or less with respect to the liquid composition under Condition II.

The content of tyrosine in the pharmaceutical composition of the present invention can be controlled such that it is within the above-described predetermined values through, for example, a method in which a step of accommodating a liquid composition in which a packaging material is filled with the liquid composition and is sealed is performed under an inert gas atmosphere during production of the pharmaceutical composition, and a method in which a step of heat-sterilizing the kit obtained through the accommodation step is performed under an inert gas atmosphere.

The content of tyrosine in the pharmaceutical composition of the present invention can be measured through a method described in examples.

Buffer agents such as a phosphate buffer solution, a tris-hydrochloric acid buffer solution, an acetate buffer solution, a carbonate buffer solution, and a citrate buffer solution may be included in the liquid composition of the present invention as necessary. These buffer agents tend to contribute to stabilization of a formulation or reduction in irritation.

Other components generally used in the technical field of formulations can be included in the liquid composition of the present invention as necessary. Other components include additives generally used in a liquid, particularly in an aqueous composition, and examples of the additives include preservatives such as benzalkonium chloride, potassium sorbate, and chlorohexidine hydrochloride; stabilizers such as sodium edetate; thickeners such as hydroxyethyl cellulose and hydroxypropyl methylcellulose; isotonic agents such as sodium chloride, potassium chloride, glycerin, sucrose, and glucose; surfactants such as polysorbate 80 and polyoxyethylene hydrogenated castor oil; and pH adjusters such as hydrochloric acid and sodium hydroxide.

In addition, examples of the other components include antioxidants, suspending agents, soothing agents, preservatives, coloring agents, sweeteners, adsorbents, and gelling agents which are generally used in liquid formulations.

Examples of the antioxidant include sodium pyrosulfite, sodium hydrogen sulfite, α-tocopherol, ascorbic acid, ascorbic acid palmitate, butylhydroxyanisole, butylhydroxytoluene, monothioglycerol, potassium metabisulfite, propionic acid, propyl gallate, sodium ascorbate, sodium iodide, sodium nitrite, sodium sulfite, and sodium thiosulfate. These may be used alone or in combination of two or more thereof.

Among these, sodium pyrosulfite, sodium hydrogen sulfite, and sodium sulfite are preferable.

### (Packaging Material)

In the pharmaceutical composition of the present invention, the liquid composition containing BPA is accommodated in a packaging material. Here, the state that the liquid composition containing BPA is accommodated in a packaging material means that the packaging material contains the liquid composition containing BPA and is sealed. The packaging material of the present invention is preferably made of a thermoplastic resin. By incorporating a thermoplastic resin into the material of the packaging material, the concentration of dissolved oxygen in the liquid composition can be easily reduced through heat-sterilizing under an inert gas atmosphere during production of the pharmaceutical composition to be described below. It is thought that this is because inert gas molecules permeate through the packaging material under an inert gas atmosphere and oxygen molecules in the liquid composition in the packaging material are replaced with the inert gas molecules.

The packaging material may be a single-layer body composed of a single layer, or may be a layered body composed of two or more layers.

The packaging material may be bag, soft bag, container, package, packet, or pouch.

The thermoplastic resin used as the material of the packaging material is not particularly limited, but examples thereof include polyethylene resin (which is also referred to as PE and includes high density polyethylene, medium density polyethylene, and low density polyethylene), polypropylene resin (also referred to as PP), polyvinyl chloride resin (also referred to as PVC), polyvinylidene chloride resin, polystyrene resin (also referred to as PS), polyvinyl acetate resin (also referred to as PVAc), polyurethane resin (PUR), polytetrafluoroethylene resins (PTFE), acrylonitrile butadiene styrene resin, and acrylic resins. A single thermoplastic resin may be included, or two or more kinds thereof may be mixed with each other. Among the above-described thermoplastic resins, polyethylene resin, polypropylene resin, and polyvinyl chloride resin are preferable.

The film thickness of the packaging material is not particularly limited, but is generally within a range of 0.001 mm or more and 1 mm or less, preferably 0.010 mm or more and 1 mm or less, and more preferably 0.100 mm or more and 1 mm or less. By setting the film thickness to be 0.001 mm or more and 1 mm or less, the liquid composition which is a content of the packaging material can be safely protected.

The oxygen permeability of the packaging material is not particularly limited, but is generally within a range of greater than 0 cm³/(m²•24h•atm) and 10,000 cm³/(m²•24h•atm) or less, preferably greater than 0 cm³/(m²•24h•atm) and 1,000 cm³/(m²•24h•atm) or less, and more preferably 100 cm³/(m²•24h•atm) or more and 1,000 cm³/(m²•24h•atm) or less. By setting the oxygen permeability to be greater than 0 cm³/(m²•24h•atm), dissolved oxygen in the liquid composition can be removed from the system (outside the packaging material) in the heat-sterilizing step during the production of the pharmaceutical composition, and therefore, the concentration of dissolved oxygen tends to be reduced. In addition, by setting the oxygen permeability to be 10,000 cm³/(m²•24h•atm) or less, oxygen tends to be able to be prevented from permeating into the packaging material from the outside during storage of the pharmaceutical composition.

The shape of the packaging material may be appropriately selected depending on administration forms of formulations, and may be, for example, a bag shape, a box shape, and a bottle shape. Among these, a bag shape is preferable as the shape of the packaging material. In addition, the packaging material may be specifically a packaging material for an infusion bag or a prefilled syringe. Among these, the packaging material is preferably for an infusion bag.

### (Method for Producing Pharmaceutical Composition) (Reference embodiment)

The production method of the present disclosure is a method for producing a pharmaceutical composition according to the present invention, which contains a liquid composition containing BPA and is accommodated in a packaging material as described above, and comprises a step I of accommodating the liquid composition containing BPA in the packaging material and a step II of heat-sterilizing the liquid composition accommodated in the packaging material under an inert gas atmosphere.

The liquid composition containing BPA in the above-described step I can be prepared through self-preparation or by obtaining a commercially available product. In the case of preparing the liquid composition containing BPA, it can be prepared by adding BPA to a solvent and adding additives such as an antioxidant or a solubilizing agent such as sorbitol thereto as necessary.

For example, in a case where a liquid composition containing BPA has a predetermined pH, for example, a pH of 6.5 or more and 8.0 or less, examples of a method for producing the liquid composition containing BPA include a method for adding a pH adjuster such as sodium hydroxide, water, BPA, and/or pharmaceutically acceptable salts of BPA to a solvent, followed by adding additives such as an antioxidant or a solubilizing agent such as sorbitol as necessary and adjusting the pH with hydrochloric acid or the like as necessary.

Examples of the pharmaceutically acceptable salts of BPA include a salt with an organic acid, a salt with an inorganic acid, a salt with an organic base, and a salt with an inorganic base.

Examples of the salt with an organic acid include acetate, trifluoroacetate, fumarate, maleate, lactate, tartrate, citrate, and methanesulfonate.

Examples of the salt with an inorganic acid include hydrochloride, sulfate, nitrate, hydrobromide, and phosphate.

Examples of the salt with an organic base include a salt with triethanolamine. Examples of the salt with an inorganic base include ammonium salt, sodium salt, potassium salt, calcium salt, and magnesium salt.

In the above-described step I, the liquid composition containing BPA is accommodated in a packaging material by filling the packaging material with the liquid composition and subsequently sealing the packaging material. At this time, the packaging material may be completely filled with the liquid composition, or a void portion may be partially present in the system together with the liquid composition.

The above-described step I is preferably performed under an inert gas atmosphere in order to further reduce the amount of dissolved oxygen of the liquid composition in the pharmaceutical composition of the present invention. Examples of the method for performing the step I under an inert gas atmosphere include a method in which a liquid composition is bubbled with inert gas during preparation of the liquid composition and/or until the liquid composition is sealed in a packaging material.

In the above-described step II, the liquid composition which has been accommodated in the packaging material and obtained in the step I is heat-sterilized. In the step II, the heat-sterilization is performed under an inert gas atmosphere. The heat-sterilization under an inert gas atmosphere may be performed such that, for example, the liquid composition which has been accommodated in the packaging material and obtained in the step I is disposed in an autoclave device or the like and the system in which the heat-sterilization is to be performed is filled with inert gas, as will be described below. In the method for filling the system, for which heat-sterilization is to be performed, with inert gas to perform the heat-sterilization, the liquid composition accommodated in the packaging material is separated from the external system via the packaging material. However, it is considered that, for example, inert gas permeates through the packaging material by filling the autoclave device with inert gas and performing heat-sterilization to replace oxygen in the packaging material with inert gas molecules, and the amount of dissolved oxygen is reduced due to dissolved oxygen in the packaging material being released outside the packaging material.

The temperature during the heat-sterilization may be appropriately adjusted, but is, from the viewpoint of performing sufficient sterilization, preferably within a range of 95°C or more and 140°C or less, preferably within a range of 100°C or more and 130°C or less, and preferably within a range of 105°C or more and 125°C or less.

The pressure during the heat-sterilization may be appropriately adjusted, but is, from the viewpoint of performing sufficient sterilization, generally preferably within a range of greater than 1 atm and 2 atm or less.

The time for the heat-sterilization may be appropriately adjusted, but is, from the viewpoint of performing sufficient sterilization, preferably 1 minute or more and 60 minutes or less, more preferably 5 minutes or more and 60 minutes or less, and still more preferably 5 minutes or more and 30 minutes or less.

Examples of inert gas in the production method of the present invention include helium, nitrogen, and argon.

The heat-sterilization under an inert gas atmosphere in the step II can be performed by an autoclave. A method for bringing an object into contact with high-pressure steam can be suitably used for heat-sterilization in the step II. Specific examples of the method for bringing an object into contact with high-pressure steam include an indirect heating method (a method of introducing hot water into a system, in which heat-sterilization is to be performed, to spray or shower the hot water onto the object and to simultaneously generate high-pressure steam) and a steam injection method (a method of introducing high-pressure steam into a system in which heat-sterilization is to be performed).

In the heat-sterilization in which an object is brought into contact with high-pressure steam, a commercially available autoclave device may be used. For example, a sterilizer manufactured by HISAKA WORKS, LTD. can be used as the autoclave device. An RCS series or a GPS series manufactured by HISAKA WORKS, LTD. can be used as the sterilizer, and the size of the device (RCS-40 to 260/10 to 120 or GPS-40 to 260/10 to 120) may be appropriately selected depending on the scale of heat-sterilization or the number of objects (a liquid composition accommodated in a packaging material) to be used for heat-sterilization. In addition, hot water spray type (SP type) device or a hot water shower type (SW type) device may be appropriately selected.

### [Examples]

Hereinafter, the present invention will be further described with reference to examples, but is not limited to the following examples.

### (Additives)

The following two kinds of antioxidants were used.

### Sodium pyrosulfite (manufactured by FUJIFILM Wako Pure Chemical Corporation) Sodium hydrogen sulfite (manufactured by FUJIFILM Wako Pure Chemical Corporation)

### (Packaging Material)

The following four kinds of packaging materials were used. These packaging materials have bag shapes, and the sizes are as shown in the following table 1.

**[Table 1]**

| Reference numeral | Material | Film thickness | Oxygen permeability | Manufacturer | Size |
|---|---|---|---|---|---|
| A | PP | 0.26 mm | 815.0 cm³/(m²•24h•atm) | Bespack co., Ltd. | 18 cm long × 9 cm wide |
| B | PE | 0.26 mm | 854.0 cm³/(m²•24h•atm) | Bespack co., Ltd. | 18 cm long × 9 cm wide |
| C | PE | 0.36 mm | 603.0 cm³/(m²•24h•atm) | Bespack co., Ltd. | 20 cm long × 12 cm wide |
| D | Heat-resistant PE | 0.26 mm | 791.0 cm³/(m²•24h•atm) | Bespack co., Ltd. | 18 cm long × 9 cm wide |

### (Examples 1 to 22, Comparative Examples 1 to 11)

### (Step of Preparing Liquid Composition)

780.0 g of L-BPA and 819.0 g of D-sorbitol were added to and dissolved in 22 L of water in which 162.2 g of sodium hydroxide was dissolved in advance. 1 mol/L hydrochloric acid was added thereto at room temperature, the pH of the mixture was adjusted to 7.6, and the volume of the mixture was adjusted to 25 L by adding water thereto to prepare a liquid composition.

In addition, when preparing the liquid composition, 5.0 g of sodium pyrosulfite or 5.0 g of sodium hydrogen sulfite was added as an antioxidant.

### (Filling Step)

Each of the packaging materials of the above-described Table 1 was filled with 95 mL of the drug solution prepared as described above while performing or not performing nitrogen bubbling, and was sealed to obtain bags containing the liquid composition.

### (Sterilization Step)

Sterilization of the above-described bags was performed. The sterilization step was performed through an indirect heating method with a hot water spray type sterilizer (RCS-120/20RSPXG) manufactured by HISAKA WORKS, LTD. The sterilization was performed for 20 minutes at 112°C or 117°C.

### (Stability Evaluation)

The stability evaluation was performed by mainly using the following model or conditions as standard conditions for a severe stability test for a pharmaceutical product based on the ICH guidelines. Specifically, as a storage test, the bags were stored for 2 weeks in a device LH21-13M (manufactured by Nagano Science Co., Ltd.) at 40°C/75%RH.

### (Concentration of Dissolved Oxygen)

The concentrations of dissolved oxygen in the liquid compositions were measured before and after the sterilization step.

Specifically, 30 mL of each of the liquid compositions in the kits was collected, and the concentrations (ppm) of dissolved oxygen were measured with a device OM-71 (manufactured by HORIBA, Ltd.)

### (Amount of Tyrosine)

The amount of tyrosine in the kits after the sterilization step and the amount of tyrosine in the kits stored at 40°C/75%RH after the sterilization step were measured. Specifically, the amount of tyrosine in a sample obtained by collecting 1 mL of each of the liquid compositions in the kits and diluting the collected liquid composition to 100 mL was measured with a high performance liquid chromatograph (Nexera X2 series, manufactured by Shimadzu Corporation).

The measurement conditions by HPLC were as follows.
Column used: Mightysil RP-18GP (5 µm, 4.6 × 150 mm) manufactured by Kanto Chemical Co., Inc.
Mobile phase: 0.05 mol/L sodium dihydrogen phosphate test solution (pH of 2.5) / methanol (95:5)
Diluted solution: 0.05 mol/L sodium dihydrogen phosphate test solution (pH of 2.0)
Column temperature: constant temperature around 40°C
Flow rate: about 0.8 mL/minute
Injection amount: 10 µL
Detection wavelength: 223 nm

### (Coloration)

The infusion bags were visually observed to check the presence or absence of coloration.

The conditions such as the types of packaging materials, the presence or absence and the types of additives, and the atmosphere were changed to obtain pharmaceutical compositions of Examples and Comparative Examples. The conditions of Examples and Comparative Examples, the characteristics of the pharmaceutical compositions obtained through Examples and Comparative Examples, and the evaluation results were shown in Tables 2 and 3.

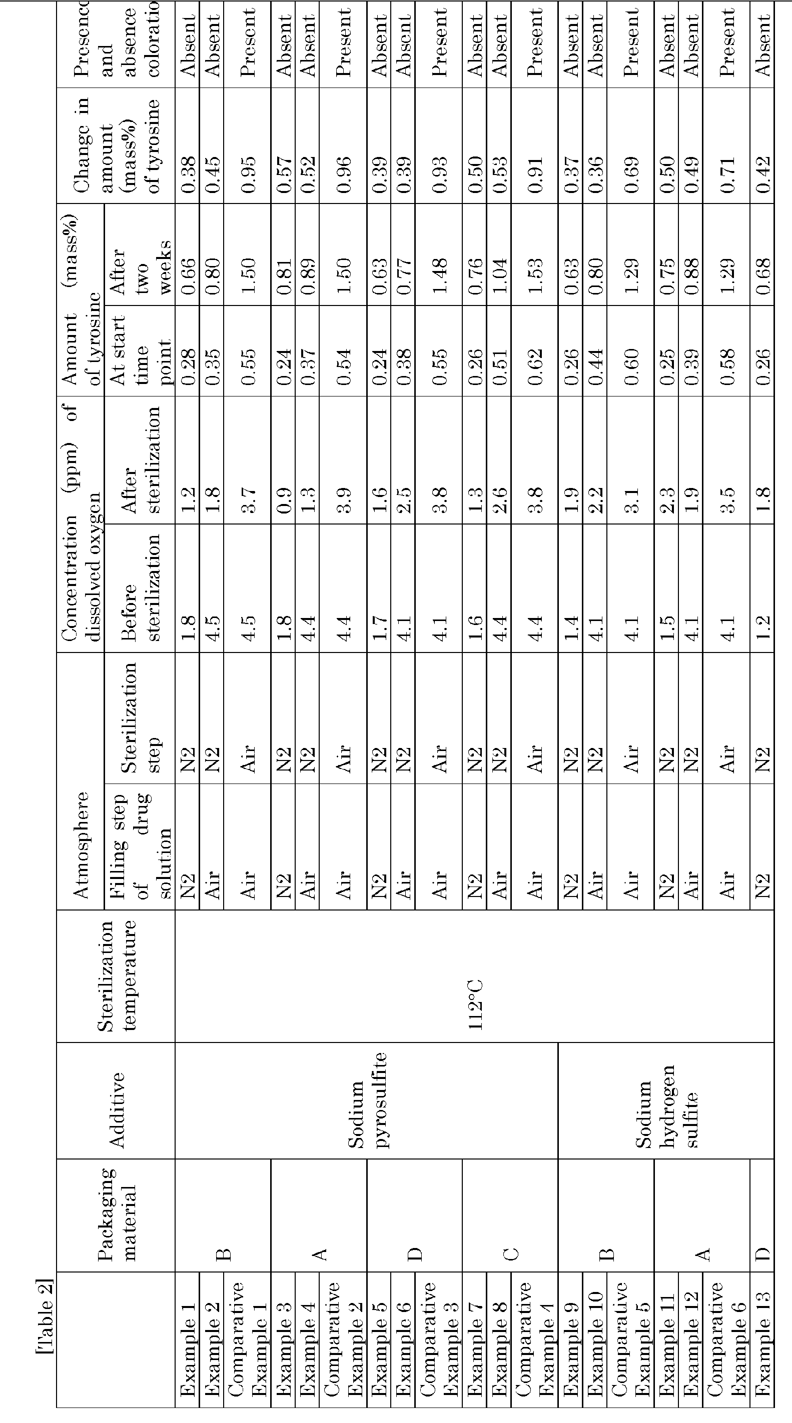

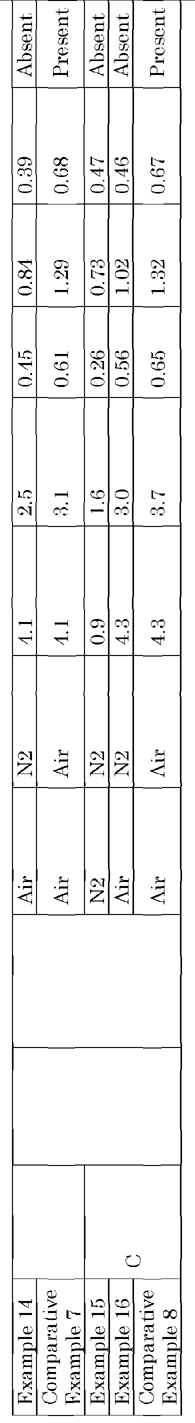

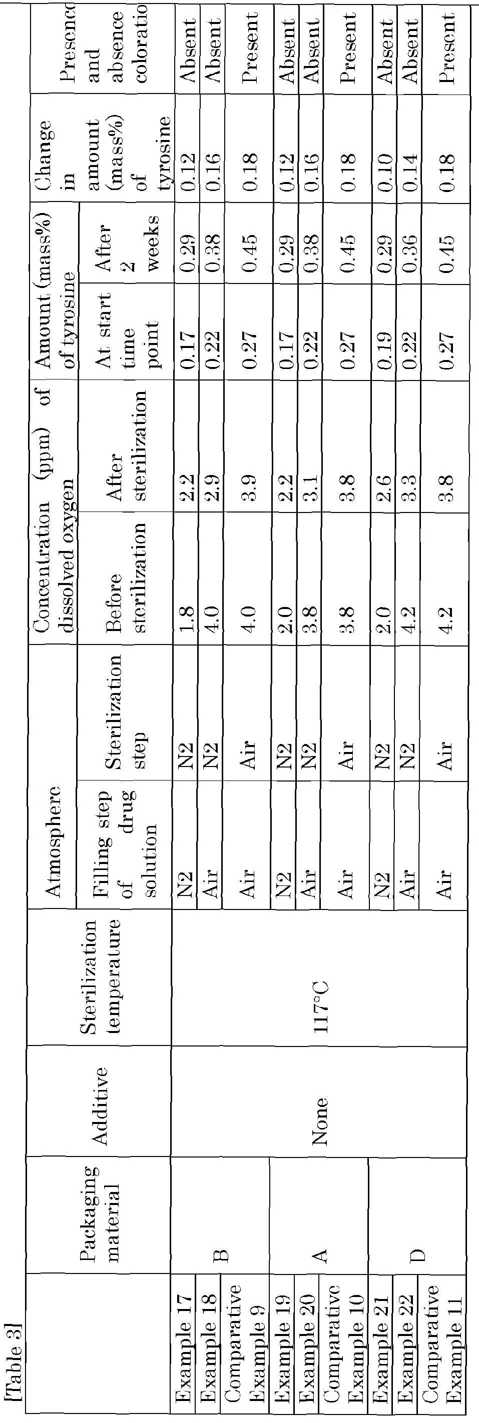

## Claims

1. A pharmaceutical composition comprising a liquid composition containing p-boronophenylalanine, wherein the liquid composition is accommodated in a packaging material, and satisfies the following Condition I or Condition II:
Condition I: the liquid composition comprises components of antioxidant less than 0.001 W/V% and a concentration of dissolved oxygen in the liquid composition is 3.5 ppm or less;
Condition II: the liquid composition comprises an antioxidant and a concentration of dissolved oxygen in the liquid composition is 3.0 ppm or less.

2. The pharmaceutical composition according to claim 1, wherein the liquid composition further comprises 0.75 mass% or less of tyrosine.

3. The pharmaceutical composition according to claim 1, wherein a tyrosine content in the liquid composition after the pharmaceutical composition is stored at 40°C/75%RH for 2 weeks is 0.40 mass% or less in the case of Condition I or 1.20 mass% or less in the case of Condition II.

4. The pharmaceutical composition according to any one of claims 1 to 3, wherein the antioxidant comprises one or more selected from the group consisting of sodium pyrosulfite, sodium hydrogen sulfite, α-tocopherol, ascorbic acid, ascorbyl palmitate,
butylhydroxyanisole, butylhydroxytoluene, monothioglycerol, potassium metabisulfite, propionic acid, propyl gallate, sodium ascorbate, sodium iodide, sodium nitrite, sodium sulfite, and sodium thiosulfate.

5. The pharmaceutical composition according to any one of claims 1 to 4, wherein the packaging material is a bag-shaped packaging material.

6. The pharmaceutical composition according to any one of claims 1 to 5, wherein a material of the packaging material comprises a thermoplastic resin, wherein the thermoplastic resin preferably comprises one or more selected from the group consisting of polyethylene resins, polypropylene resins, polyvinyl chloride resins, polyvinylidene chloride resins, polystyrene resins, polyvinyl acetate resins, polyurethane resins, polytetrafluoroethylene resins, acrylonitrile butadiene styrene resins, and acrylic resins.

7. The pharmaceutical composition according to any one of claims 1 to 6, wherein the packaging material is an infusion bag.

## Patentansprüche

1. Pharmazeutische Zusammensetzung umfassend eine flüssige Zusammensetzung enthaltend p-Boronophenylalanin, wobei die flüssige Zusammensetzung in einem Verpackungsmaterial untergebracht ist und die folgende Bedingung I oder Bedingung II erfüllt:
Bedingung I: die flüssige Zusammensetzung umfasst Komponente von Antioxidans von weniger als 0,001 W/V% und die Konzentration an gelöstem Sauerstoff in der flüssigen Zusammensetzung beträgt 3,5 ppm oder weniger;
Bedingung II: die flüssige Zusammensetzung umfasst ein Antioxidans und die Konzentration an gelöstem Sauerstoff in der flüssigen Zusammensetzung beträgt 3,0 ppm oder weniger.

2. Pharmazeutische Zusammensetzung gemäß Anspruch 1, wobei die flüssige Zusammensetzung ferner 0,75 Massen-% oder weniger Tyrosin umfasst.

3. Pharmazeutische Zusammensetzung gemäß Anspruch 1, wobei der Tyrosingehalt in der flüssigen Zusammensetzung, nachdem die pharmazeutische Zusammensetzung 2 Wochen lang bei 40°C/75% RH gelagert wurde, 0,40 Massen-% oder weniger im Fall von Bedingung I oder 1,20 Massen-% oder weniger im Fall von Bedingung II beträgt.

4. Pharmazeutische Zusammensetzung gemäß mindestens einem der Ansprüche 1 bis 3, wobei das Antioxidans umfasst eines oder mehrere, ausgewählt aus der Gruppe bestehend aus Natriumpyrosulfit, Natriumhydrogensulfit, α-Tocopherol, Ascorbinsäure, Ascorbylpalmitat,
Butylhydroxyanisol, Butylhydroxytoluol, Monothioglycerin, Kaliummetabisulfit, Propionsäure, Propylgallat, Natriumascorbat, Natriumjodid, Natriumnitrit, Natriumsulfit und Natriumthiosulfat.

5. Pharmazeutische Zusammensetzung gemäß mindestens einem der Ansprüche 1 bis 4, wobei das Verpackungsmaterial ein beutelförmiges Verpackungsmaterial ist.

6. Pharmazeutische Zusammensetzung gemäß mindestens einem der Ansprüche 1 bis 5, wobei ein Material des Verpackungsmaterials ein thermoplastisches Harz umfasst, wobei das thermoplastische Harz vorzugsweise eines oder mehrere umfasst, ausgewählt aus der Gruppe, bestehend aus Polyethylenharze, Polypropylenharze, Polyvinylchloridharze, Polyvinylidenchloridharze, Polystyrolharze, Polyvinylacetatharze, Polyurethanharze, Polytetrafluorethylenharze, Acrylnitril-Butadien-Styrolharze und Acrylharze.

7. Pharmazeutische Zusammensetzung gemäß mindestens einem der Ansprüche 1 bis 6, wobei das Verpackungsmaterial ein Infusionsbeutel ist.

## Revendications

1. Composition pharmaceutique comprenant une composition liquide contenant de la p-boronophénylalanine, dans laquelle la composition liquide est logée dans un matériau d'emballage, et satisfait la Condition I ou la Condition II suivante :
Condition I : la composition liquide comprend des composants d'antioxydant à raison de moins de 0,001 % en p/v et une concentration d'oxygène dissous dans la composition liquide est de 3,5 ppm ou moins ;
Condition II : la composition liquide comprend un antioxydant et une concentration d'oxygène dissous dans la composition liquide est de 3,0 ppm ou moins.

2. Composition pharmaceutique selon la revendication 1, dans laquelle la composition liquide comprend en outre 0,75 % en masse ou moins de tyrosine.

3. Composition pharmaceutique selon la revendication 1, dans laquelle une teneur en tyrosine dans la composition liquide après que la composition pharmaceutique est stockée à 40 °C/75 % d'HR pendant 2 semaines est de 0,40 % en masse ou moins dans le cas de la Condition I ou de 1,20 % en masse ou moins dans le cas de la Condition II.

4. Composition pharmaceutique selon l'une quelconque des revendications 1 à 3, dans laquelle l'antioxydant comprend un ou plusieurs sélectionnés dans le groupe consistant en pyrosulfite de sodium, hydrogénosulfite de sodium, α-tocophérol, acide ascorbique, palmitate d'ascorbyle, butylhydroxyanisole, butylhydroxytoluène, monothioglycérol, métabisulfite de potassium, acide propionique, gallate de propyle, ascorbate de sodium, iodure de sodium, nitrite de sodium, sulfite de sodium et thiosulfate de sodium.

5. Composition pharmaceutique selon l'une quelconque des revendications 1 à 4, dans laquelle le matériau d'emballage est un matériau d'emballage en forme de sac.

6. Composition pharmaceutique selon l'une quelconque des revendications 1 à 5, dans laquelle un matériau du matériau d'emballage comprend une résine thermoplastique, dans laquelle la résine thermoplastique comprend préférablement un ou plusieurs sélectionnés dans le groupe consistant en résines de polyéthylène, résines de polypropylène, résines de chlorure de polyvinyle, résines de chlorure de polyvinylidène, résines de polystyrène, résines d'acétate de polyvinyle, résines de polyuréthane, résines de polytétrafluoroéthylène, résines d'acrylonitrile butadiène styrène, et résines acryliques.

7. Composition pharmaceutique selon l'une quelconque des revendications 1 à 6, dans laquelle le matériau d'emballage est une poche de perfusion.
